(19) Europäisches Patentamt
European Patent Office
Office européen des brevets

(11) Veröffentlichungsnummer: **0 041 922**
**B1**

# (12) EUROPÄISCHE PATENTSCHRIFT

(45) Veröffentlichungstag der Patentschrift: **03.10.84**

(51) Int. Cl.³: **C 07 D 239/42** // C09B62/245, C09B62/20

(21) Anmeldenummer: **81810215.4**

(22) Anmeldetag: **03.06.81**

(54) **Verfahren zur Herstellung von aminosubstituierten 1-Amino-8-hydroxynaphthalin-sulfonsäuren.**

(30) Priorität: **09.06.80 CH 4419/80**

(43) Veröffentlichungstag der Anmeldung: **16.12.81 Patentblatt 81/50**

(45) Bekanntmachung des Hinweises auf die Patenterteilung: **03.10.84 Patentblatt 84/40**

(84) Benannte Vertragsstaaten: **CH DE FR GB LI**

(56) Entgegenhaltungen:
**EP-A-0 019 785**
**FR-A-1 545 174**
**US-A-3 822 263**
**US-A-4 007 164**

(73) Patentinhaber: **CIBA-GEIGY AG, Postfach, CH-4002 Basel (CH)**

(72) Erfinder: **Seiler, Herbert, Dr., Leimgrubenweg 60, CH-4125 Riehen (CH)**
Erfinder: **Deitz, Rolf, Herrengrabenweg 64, CH-4054 Basel (CH)**

EP 0 041 922 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Monoacylierung von 1-Amino-8-hydroxynaphthalin-sulfonsäuren in wässriger Lösung verläuft uneinheitlich, da als Konkurrenzreaktion zu der Acylierung der Aminogruppe immer – je nach Acylierungsmittel und pH-Wert in unterschiedlichem Masse – auch Acylierung der Hydroxygruppe stattfindet. Der Anteil an dem aminoacylierten Produkt ist dabei um so grösser, je mehr der pH-Wert der Reaktionsmischung in den sauren Bereich verschoben ist.

Es werden daher 1-Amino-8-hydroxynaphthalin-sulfonsäuren mit faserreaktiven Acylierungsmitteln (Reaktivkomponenten), wie Cyanurchlorid, Cyanurfluorid, Fluorchlorpyrimidin oder Trifluorpyrimidin, in stark bis schwach saurem pH-Bereich zu aminosubstituierten Verbindungen kondensiert. Verfahrensvorschriften für derartige Kondensationen sind z.B. aus den US-Patentschriften 3 822 263 und 4 007 164 bekannt.

Wir haben gefunden, dass man 1-Amino-8-hydroxynaphthalin-sulfonsäuren mit bestimmten Reaktivkomponenten in alkalischem pH-Bereich mit besseren Ausbeuten zu Aminomonokondensationsprodukten umsetzen kann als in saurem Bereich, pH $<7$.

Dieses Ergebnis ist überraschend, denn nach der geltenden Regel hätte man in alkalischem Bereich zunehmend die Bildung des unerwünschten, an der Hydroxygruppe kondensierten Produktes erwartet.

Gegenstand der Erfindung ist somit ein Verfahren zur Herstellung von Verbindungen der Formel

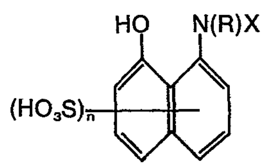

(1),

worin R Wasserstoff, Methyl oder Äthyl, X ein Mono-, Di- oder Trifluor-pyrimidinrest und $n = 1$ oder 2 ist, durch Kondensation von 1-Amino-8-hydroxynaphthalin-sulfonsäuren der Formel

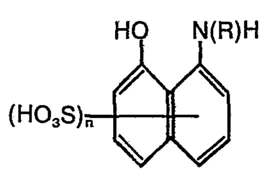

(2)

mit Di-, Tri- oder Tetrafluor-pyrimidinen in wässriger Lösung oder Suspension, dadurch gekennzeichnet, dass die Kondensation bei pH $>7$ ausgeführt wird.

Die erfindungsgemässe Umsetzung wird in wässriger Lösung oder Suspension, bei niedriger Temperatur, vorzugsweise zwischen 0 °C und Raumtemperatur, ausgeführt. Der pH-Wert der Reaktionsmischung wird durch laufende Zugabe von Alkali z.B. Natronlauge, vorzugsweise zwischen 8 und 12, gehalten.

Als Ausgangsstoffe der Formel (2) können genannt werden:

1-Amino-8-hydroxynaphthalin-3,6-disulfonsäure,
1-Amino-8-hydroxynaphthalin-4,6-disulfonsäure,
1-Amino-8-hydroxynaphthalin-4-sulfonsäure,
1-Amino-8-hydroxynaphthalin-6-sulfonsäure,
1-N-Methylamino-8-hydroxynaphthalin-3,6-disulfonsäure,
1-N-Äthylamino-8-hydroxynaphthalin-3,6-disulfonsäure,
1-N-Methylamino-8-hydroxynaphthalin-4,6-disulfonsäure,
1-N-Methylamino-8-hydroxynaphthalin-4-sulfonsäure.

Vorzugsweise verwendet man 1-Amino-8-hydroxynaphthalin-sulfonsäuren der Formel (2), worin R Wasserstoff ist, als Ausgangsverbindungen.

Als Di- und Trifluor-pyrimidine kommen sowohl Pyrimidine, die ausschliesslich Fluoratome als Ringsubstituenten enthalten, als auch Pyrimidine, die ausser den Fluoratomen weitere abspaltbare oder nichtabspaltbare Ringsubstituenten enthalten, in Betracht. Derartige abspaltbare oder nichtabspaltbare Substituenten sind z.B. Chlor, Brom, Nitro, Cyan, Carbonamido, Methylsulfonyl, Äthylsulfonyl und Methyl. Diese sind im Falle der Trifluorpyrimidine vorzugsweise in 5-Stellung, und im Falle der Difluorpyrimidine vorzugsweise in 5- und/oder 6- (bzw. der gleichwertigen 4-) Stellung des Pyrimidinringes gebunden.

Reaktivkomponenten der Fluorpyrimidinreihe, die erfindungsgemäss mit den 1-Amino-8-hydroxynaphthalin-sulfonsäuren der Formel (2) kondensiert werden können, sind zum Beispiel:

2,4,6-Trifluor-pyrimidin,
2,4-Difluorpyrimidin,
2,4-Difluor-6-methylpyrimidin,
2,6-Difluor-4-methyl-5-chlorpyrimidin,
2,4-Difluor-pyrimidin-5-äthylsulfon,
2,6-Difluor-4-chlorpyrimidin,
2,4,6-Trifluor-5-chlorpyrimidin,
2,6-Difluor-4-methyl-5-brompyrimidin,
2,4-Difluor-5,6-dichlor- oder -dibrompyrimidin,
4,6-Difluor-2,5-dichlor- oder -dibrompyrimidin,
2,6-Difluor-4-brompyrimidin,
2,4,6-Trifluor-5-brompyrimidin,
2,4,6-Trifluor-5-chlormethylpyrimidin,
2,4,6-Trifluor-5-nitropyrimidin,
2,4,6-Trifluor-5-cyanpyrimidin,
2,4,6-Trifluorpyrimidin-5-carbonsäurealkylester oder -5-carbonsäureamide,
2,6-Difluor-5-methyl-4-chlorpyrimidin,
2,6-Difluor-5-chlorpyrimidin,
2,4,6-Trifluor-5-methylpyrimidin,
2,4,5-Trifluor-6-methylpyrimidin,
2,4-Difluor-5-nitro-6-chlorpyrimidin,
2,4-Difluor-5-cyanpyrimidin,
2,4-Difluor-5-methylpyrimidin,
6-Trifluormethyl-5-chlor-2,4-difluor-pyrimidin,
5-Trifluormethyl-2,4,6-trifluorpyrimidin,

2,4-Difluor-5-nitro-pyrimidin,
2,4-Difluor-5-trifluormethyl-pyrimidin,
2,4-Difluor-5-methylsulfonyl-pyrimidin,
2,4-Difluor-5-phenyl-pyrimidin,
2,4-Difluor-5-carbonamido-pyrimidin,
2,4-Difluor-5-carbomethoxy-pyrimidin,
2,4-Difluor-6-trifluormethyl-pyrimidin,
2,4-Difluor-5-brom-6-trifluormethyl-pyrimidin,
2,4-Difluor-6-carbonamido-pyrimidin,
2,4-Difluor-6-carbomethoxy-pyrimidin,
2,4-Difluor-6-phenyl-pyrimidin,
2,4-Difluor-6-cyan-pyrimidin,
2,4,6-Trifluor-5-methylsulfonyl-pyrimidin,
2,4-Difluor-5-sulfonamido-pyrimidin,
2,4-Difluor-5-chlor-6-carbomethoxy-pyrimidin,
2,4,5-Trifluorpyrimidin,
2,4,5,6-Tetrafluorpyrimidin,
4,6-Difluor-5-chlorpyrimidin.

Vorzugsweise verwendet man Trifluor-pyrimidine als Ausgangsverbindungen.

Ein wertvoller Vertreter der Verbindungen der Formel (1) ist die Verbindung der Formel

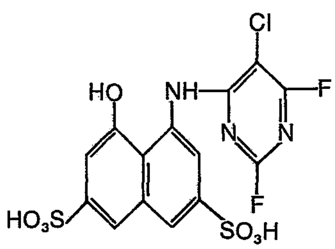

(3).

Sie wird erfindungsgemäss hergestellt, indem man 1-Amino-8-hydroxynaphthalin-3,6-disulfonsäure mit 2,4,6-Trifluor-5-chlorpyrimidin in wässriger Lösung bei einem pH-Wert von vorzugsweise 8,5 bis 10 kondensiert.

Die nach dem erfindungsgemässen Verfahren erhaltenen Verbindungen der Formel (1) sind wertvolle Zwischenverbindungen für die Herstellung von Reaktivfarbstoffen.

Zu diesem Zweck werden die erfindungsgemäss hergestellten Verbindungen der Formel (1) durch Umsetzungen mit anderen Verbindungen, die für die Herstellung von Reaktivfarbstoffen geeignet sind, z.B. aromatischen Aminen und Aminofarbstoffen, in die gewünschten Endfarbstoffe umgewandelt. Bei derartigen Umsetzungen handelt es sich vor allem um Kupplungen.

Ein Gegenstand der Erfindung ist daher auch die Verwendung von erfindungsgemäss hergestellten Verbindungen der Formel (1) zur weiteren Umsetzung mit Farbstoffvorprodukten.

Eine bevorzugte Ausführungsform dieser Verwendung ist dadurch gekennzeichnet, dass man die Verbindungen der Formel (1) mit diazotierten Diazokomponenten kuppelt.

Geeignete Diazokomponenten sind in grosser Zahl bekannt. Die Diazotierung der eine diazotierbare Aminogruppe enthaltenden Diazokomponenten erfolgt in der Regel durch Einwirkung salpetriger Säure in wässrig-mineralsaurer Lösung bei tiefer Temperatur, die Kupplung bei schwach sauren, neutralen bis schwach alkalischen pH-Werten.

Besonders interessante Azofarbstoffe werden durch Kupplung der Verbindung der Formel (3) mit diazotierten Diazokomponenten erhalten.

Ein besonders wertvoller roter Monoazofarbstoff wird hergestellt, indem man die Verbindung der Formel (3) mit diazotierter 2-Aminonaphthalin-1,5-disulfonsäure kuppelt.

Ein weiterer wertvoller Monoazofarbstoff wird hergestellt, indem man die Verbindung der Formel (3) mit diazotierter 1-Aminobenzol-2-sulfonsäure kuppelt.

Die in der oben beschriebenen Weise durch Kupplung erhaltenen Reaktivfarbstoffe können isoliert und zu brauchbaren, trockenen Färbepräparaten verarbeitet werden. Die Isolierung erfolgt bei Raumtemperatur durch Aussalzen und Filtrieren. Die filtrierten Farbstoffe können gegebenenfalls nach Zugabe von Coupagemitteln und/oder Puffermitteln, z.B. nach Zugabe eines Gemisches gleicher Teile Mono- und Dinatriumphosphat, getrocknet werden; vorzugsweise wird die Trocknung bei nicht zu hohen Temperaturen und unter vermindertem Druck vorgenommen. Durch Zerstäubungstrocknung des ganzen Herstellungsgemisches kann man in gewissen Fällen die erfindungsgemässen trockenen Präparate direkt, d.h. ohne Zwischenisolierung der Farbstoffe, herstellen.

Die Reaktivfarbstoffe zeichnen sich durch eine hohe Reaktivität, einen hohen Fixiergrad, gutes Aufbauvermögen und eine gute Auswaschbarkeit der nicht fixierten Anteile aus. Die mit den Reaktivfarbstoffen erhältlichen Färbungen und Drucke sind farbstark und haben eine gute Lichtechtheit sowie gute Nassechtheiten, z.B. eine gute Waschechtheit.

Sie eignen sich zum Färben und Bedrucken der verschiedensten Materialien, wie Seide, Leder, Wolle, Superpolyamidfasern und Superpolyurethanen, insbesondere aber cellulosehaltiger Materialien faseriger Struktur, wie Leinen, Zellstoff, regenerierte Cellulose und vor allem Baumwolle. Sie eignen sich sowohl für das Ausziehverfahren als auch zum Färben nach den Foulardfärbeverfahren, wonach die Ware mit wässrigen und gegebenenfalls auch salzhaltigen Farbstofflösungen imprägniert wird und die Farbstoffe nach einer Alkalibehandlung oder in Gegenwart von Alkali, gegebenenfalls unter Wärmeeinwirkung, fixiert werden.

Sie eignen sich auch zum Druck, insbesondere auf Baumwolle, ebenso aber auch zum Bedrucken von stickstoffhaltigen Fasern, z.B. von Wolle, Seide oder Wolle enthaltenden Mischgeweben.

Zwecks Verbesserung der Nassechtheiten empfiehlt es sich, die Färbungen und Drucke einem gründlichen Spülen mit kaltem und heissem Wasser, gegebenenfalls unter Zusatz eines dispergierend wirkenden und die Diffusion der nicht fixierten Anteile fördernden Mittels zu unterwerfen.

In den nachfolgenden Beispielen sind die Teile, sofern nicht anders angegeben, Gewichtsteile,

und die Temperaturen sind in Celsiusgraden angegeben.

Beispiel 1

31,3 g 1-Amino-8-hydroxynaphthalin-3,6-disulfonsäure werden in 500 ml Wasser neutral gelöst. Nach Abkühlung auf 10 bis 15 °C lässt man 16,8 g 2,4,6-Trifluor-5-chlorpyrimidin in 30 Minuten zutropfen. Der pH-Wert der Lösung wird mittels 2n Natronlauge bei 10 gehalten, bis die Umsetzung beendet ist.

30,3 g 2-Aminonaphthalin-1,5-disulfonsäure werden bei pH 6 in 300 ml Wasser gelöst. Man kühlt durch Zugabe von Eis auf 0 bis 5 °C, versetzt die Lösung mit 25 ml konz. Salzsäure und diazotiert durch Zutropfen von 25 ml 4n Natriumnitritlösung. Nach einstündigem Nachrühren bei 0 bis 5 °C wird überschüssiges Nitrit mit Amidosulfonsäure zerstört.

Die Diazoniumverbindung wird nun zur Lösung der oben hergestellten Kupplungskomponente langsam zugegeben und der pH-Wert in der Kupplungslösung mit 2n Natronlauge bei 7,5 bis 8 gehalten. Nach beendeter Kupplung lässt man die Temperatur auf 20 °C ansteigen und salzt den Farbstoff der Formel

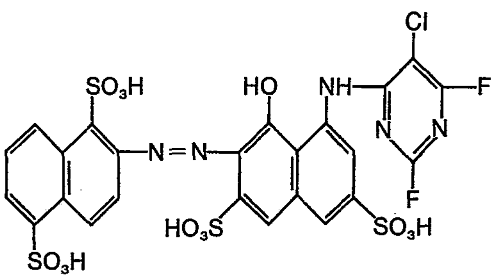

mit Natriumchlorid aus, saugt ihn ab, wäscht mit 15%iger Natriumchloridlösung und trocknet bei 40 °C im Vakuum. Man erhält 78,5 g eines dunkelroten Farbstoffpulvers, das Baumwolle und Regeneratcellulose in blaustichig roten Tönen färbt.

Kondensiert man 31,3 g 1-Amino-8-hydroxynaphthalin-3,6-disulfonsäure mit 16,8 g 2,4,6-Trifluor-5-chlorpyrimidin statt bei pH 10 bei einem pH-Wert von 5, verfährt aber sonst wie in Beispiel 1 angegeben, so erhält man nur 65,3 g Farbstoff der gleichen koloristischen Stärke.

Ersetzt man im Beispiel 1 die 1-Amino-8-hydroxynaphthalin-3,6-disulfonsäure durch äquivalente Mengen 1-Amino-8-hydroxynaphthalin-4,6-disulfonsäure
oder
1-Amino-8-hydroxynaphthalin-4-sulfonsäure
oder
1-Amino-8-hydroxynaphthalin-6-sulfonsäure
und verwendet anstelle der 2-Naphthylamin-1,5-disulfonsäure äquivalente Mengen einer der im folgenden genannten Diazokomponenten und verfährt im übrigen wie beschrieben, so erhält man ebenfalls höhere Farbstoffausbeuten, als wenn man bei einem pH-Wert von 5 kondensiert.

Diazokomponenten:
1-Aminobenzol-2-sulfonsäure,
1-Aminobenzol-4-sulfonsäure,
1-Aminobenzol-4-carbonsäure,
1-Aminobenzol-2,4-disulfonsäure,
1-Aminobenzol-2,5-disulfonsäure,
1-Amino-4-methylbenzol-2-sulfonsäure,
1-Amino-2-methylbenzol-5-sulfonsäure,
1-Amino-2-methylbenzol-4-sulfonsäure,
1-Amino-2-methylbenzol-4,6-disulfonsäure,
1-Amino-2,4-dimethylbenzol-5-sulfonsäure,
1-Amino-2,4-dimethylbenzol-6-sulfonsäure,
1-Amino-4-chlorbenzol-2-sulfonsäure,
1-Amino-2-chlorbenzol-5-sulfonsäure,
1-Amino-2-chlorbenzol-4-sulfonsäure,
1-Amino-3-chlorbenzol-2-sulfonsäure,
1-Amino-3,4-dichlorbenzol-6-sulfonsäure,
1-Amino-2,5-dichlorbenzol-4-sulfonsäure,
1-Amino-5-methyl-4-chlorbenzol-2-sulfonsäure,
1-Amino-4-methyl-5-chlorbenzol-2-sulfonsäure,
1-Amino-2-carboxybenzol-4-sulfonsäure,
1-Amino-2-carboxybenzol-5-sulfonsäure,
1-Amino-4-carboxybenzol-2-sulfonsäure,
1-Amino-5-carboxybenzol-2-sulfonsäure,
1-Amino-4-methoxybenzol-2-sulfonsäure,
1-Amino-4-äthoxybenzol-2-sulfonsäure,
1-Amino-2-methoxybenzol-5-sulfonsäure,
1-Amino-4-methoxybenzol-2,5-disulfonsäure,
1-Amino-3-acetylaminobenzol-6-sulfonsäure,
1-Amino-4-acetylaminobenzol-2-sulfonsäure,
2-Naphthylamin-1-sulfonsäure,
2-Naphthylamin-5-sulfonsäure,
2-Naphthylamin-6-sulfonsäure,
2-Naphthylamin-7-sulfonsäure,
2-Naphthylamin-8-sulfonsäure,
2-Naphthylamin-1,7-disulfonsäure,
2-Naphthylamin-3,6-disulfonsäure,
2-Naphthylamin-4,8-disulfonsäure,
2-Naphthylamin-5,7-disulfonsäure,
2-Naphthylamin-6,8-disulfonsäure,
2-Naphthylamin-1,5,7-trisulfonsäure,
2-Naphthylamin-3,6,8-trisulfonsäure,
2-Naphthylamin-4,6,8-trisulfonsäure,
1-Naphthylamin-4-sulfonsäure,
1-Naphthylamin-5-sulfonsäure,
1-Naphthylamin-6-sulfonsäure,
1-Naphthylamin-7-sulfonsäure,
1-Naphthylamin-8-sulfonsäure,
1-Naphthylamin-3,6-disulfonsäure,
1-Naphthylamin-3,7-disulfonsäure,
1-Naphthylamin-3,8-disulfonsäure,
1-Naphthylamin-4,6-disulfonsäure,
1-Naphthylamin-4,7-disulfonsäure,
1-Naphthylamin-4,8-disulfonsäure,
1-Naphthylamin-5,7-disulfonsäure,
1-Naphthylamin-6,8-disulfonsäure,
1-Naphthylamin-2,4,6-trisulfonsäure,
1-Naphthylamin-3,6,8-trisulfonsäure,
1-Naphthylamin-4,6,8-trisulfonsäure.

Weitere wertvolle erfindungsgemässe Farbstoffe werden erhalten, wenn man anstelle der 16,8 g 2,4,6-Trifluor-5-chlor-pyrimidin äquivalente Mengen einer der im folgenden genannten Acylierungskomponenten verwendet:
2,4,5,6-Tetrafluorpyrimidin,

2,4,6-Trifluorpyrimidin,
2,4,6-Trifluor-5-methylsulfonyl-pyrimidin,
2,4,6-Trifluor-5-cyan-pyrimidin,
2,4-Difluor-5-cyan-pyrimidin,
2,4-Difluor-5-chlor-6-methyl-pyrimidin,
2,4-Difluor-5-chlor-pyrimidin,
4,6-Difluor-5-chlor-pyrimidin.

Beispiel 2

Wird Baumwolle mit einer Lösung, die pro 1000 ml Wasser 20 g des Farbstoffs, erhalten gemäss Beispiel 1, 10 g Natriumbicarbonat und 100 g Harnstoff enthält, foulardiert, während 30 Sekunden einer Trockenhitze von 140 °C ausgesetzt und anschliessend während 10 Minuten kochend geseift, so erhält man eine farbstarke, brillante, blaustichig rote Färbung, die sehr licht- und nassecht ist.

Beispiel 3

Wird Baumwolle mit einer Lösung, die pro 1000 ml Wasser 20 g des Farbstoffes, erhalten gemäss Beispiel 1, 20 g Natriumbicarbonat und 50 g Natriumchlorid enthält, foulardiert, aufgerollt, während 4 Stunden gelagert und anschliessend während 10 Minuten kochend geseift, so erhält man eine tiefe, reine, blaustichig rote Färbung, die nach dem Seifen sehr gut licht- und nassecht ist.

Beispiel 4

2 g des nach Beispiel 1 erhaltenen Farbstoffes werden in 5000 ml Wasser bei 40 °C gelöst und anschliessend mit 0,5 g eines Kondensationsproduktes aus 25 Mol Äthylenoxid und 1 Mol Octadecylalkohol oder Octadecylamin, 6 g Essigsäure und schliesslich mit 0,5 g einer polyquaternären Ammoniumverbindung, beispielsweise des Kondensationsproduktes aus 11,5 g N,N',N''-Pentamethyldiäthylentriamin und 14,3 g β,β'-Dichlordimethyläther, versetzt. Man geht in das so erhaltene Färbebad mit 100 g Wolle ein, erwärmt es innerhalb 30 Minuten zum Kochen und färbt während einer Stunde bei dieser Temperatur. Anschliessend wird mit Wasser von 60 °C gespült. Man erhält ohne alkalische Nachbehandlung eine gleichmässige, blaustichig rote Färbung von guten Echtheiten.

Beispiel 5

Mit einer Druckpaste bestehend aus

30 g des Farbstoffes gemäss Beispiel 1,
200 g Harnstoff,
400 g Wasser,
340 g einer 5%igen wässrigen Lösung von Natriumalginat, und
30 g Natriumcarbonat,

bedruckt man Baumwollgewebe, dämpft die bedruckte Ware während 30 Sekunden mit Sattdampf bei einer Temperatur von ca. 105 °C, wäscht die Ware zuerst kalt und dann heiss, spült während 15 Minuten mit einer Seifenlösung (5 g/l Seife) bei Kochtemperatur, wäscht abermals heiss und kalt und trocknet.

Man erhält ein rein blaustichig rot bedrucktes Baumwollgewebe, welches eine sehr gute Licht- und Nassechtheit aufweist.

Verwendet man anstelle des Baumwollgewebes ein Zellwollgewebe und dämpft nicht während 30 Sekunden die bedruckte Ware, sondern fixiert während 1 Minute bei 110 bis 150 °C, und arbeitet im übrigen analog der Angabe des Beispiels 5, so erhält man ein bedrucktes Zellwollgewebe, welches die gleich wertvollen Eigenschaften aufweist.

## Patentansprüche

1. Verfahren zur Herstellung von Verbindungen der Formel

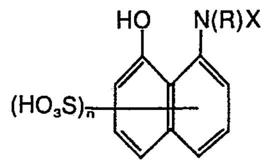

(1),

worin R Wasserstoff, Methyl oder Äthyl, X ein Mono-, Di- oder Trifluor-pyrimidinrest und n = 1 oder 2 ist, durch Kondensation von 1-Amino-8-hydroxynaphthalin-sulfonsäuren der Formel

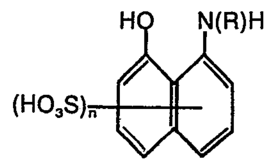

(2)

mit Di-, Tri- oder Tetrafluor-pyrimidinen in wässriger Lösung oder Suspension, dadurch gekennzeichnet, dass die Kondensation bei pH > 7 ausgeführt wird.

2. Verfahren gemäss Anspruch 1, dadurch gekennzeichnet, dass man 1-Amino-8-hydroxynaphthalin-sulfonsäuren der Formel (2), worin R Wasserstoff ist, als Ausgangsverbindungen verwendet.

3. Verfahren gemäss Anspruch 1 oder 2, dadurch gekennzeichnet, dass man Trifluor-pyrimidine als Ausgangsverbindungen verwendet.

4. Verfahren gemäss Anspruch 3, dadurch gekennzeichnet, dass man 1-Amino-8-hydroxynaphthalin-3,6-disulfonsäure mit 2,4,6-Trifluor-5-chlorpyrimidin in wässriger Lösung bei pH 8,5 bis 10 kondensiert.

## Claims

1. A process for the production of a compound of the formula

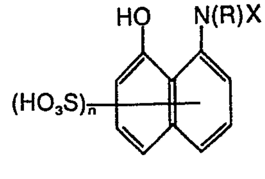

(1)

wherein R is hydrogen, methyl or ethyl, X is a monofluoropyrimidine, difluoropyrimidine or trifluoropyrimidine radical, and n is 1 or 2, which

process comprises condensing a 1-amino-8-hydroxynaphthalenesulfonic acid of the formula

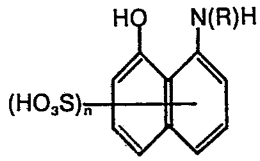

(2)

with a difluoro-, trifluoro- or tetrafluoropyrimidine, in aqueous solution or suspension, at pH >7.

2. A process according to claim 1, wherein the starting material is a 1-amino-8-hydroxynaphthalenesulfonic acid of the formula (2), wherein R is hydrogen.

3. A process according to either of claims 1 or 2, wherein the starting material is a trifluoropyrimidine.

4. A process according to claim 3, wherein 1-amino-8-hydroxynaphthalene-3,6-disulfonic acid is condensed with 2,4,6-trifluoro-5-chloropyrimidine in aqueous solution at pH 8,5 to 10.

**Revendications**

1. Procédé pour la préparation de composés de formule:

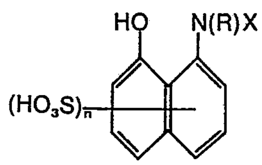

(1),

dans laquelle R est de l'hydrogène, un groupe méthyle ou éthyle; X est un reste d'une mono-, difluoro- ou trifluoro-pyrimidine et n = 1 ou 2, par condensation d'acides 1-amino-8-hydroxynaphtalènesulfoniques ayant la formule

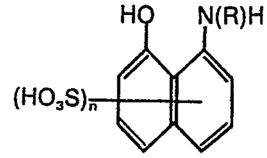

(2)

avec des difluoro-, trifluoro- ou tétrafluorpyrimidines en solution ou en suspension aqueuse, caractérisé par le fait que la condensation est effectuée à un pH supérieur à 7.

2. Procédé selon la revendication 1, caractérisé par le fait qu'on utilise comme composés de départ des acides 1-amino-8-hydroxynaphtalènesulfoniques de formule (2) dans lesquels R est de l'hydrogène.

3. Procédé selon les revendications 1 ou 2, caractérisé par le fait qu'on utilise comme composés de départ des trifluoro-pyrimidines.

4. Procédé selon la revendication 3, caractérisé par le fait qu'on condense un acide 1-amino-8-hydroxynaphtalène-3,6-disulfonique avec une 2,4,6-trifluoro-5-chloropyrimidine, en solution aqueuse, à un pH compris entre 8,5 et 10.